# EUROPÄISCHE PATENTSCHRIFT

(11) **EP 3 229 919 B1**
(45) Veröffentlichungstag und Bekanntmachung des Hinweises auf die Patenterteilung: **13.03.2024**
(21) Anmeldenummer: 15794192.3
(22) Anmeldetag: 13.11.2015
(51) Int. Cl.: A61K 8/34, A61K 8/41, A61Q 5/10

(54) **ZUSAMMENSETZUNG UND VERFAHREN ZUR BEHANDLUNG KERATINHALTIGER FASERN MIT ENTSPANNUNGSVERDAMPFUNG**
COMPOSITION AND PROCEDURE FOR TREATING KERATIN-CONTAINING FIBERS WITH FLASH EVAPORATION
COMPOSITION ET PROCEDUE POUR TRAITER LES FILAMENTS KERATINIQUES AVEC EVAPORISATION INSTANTANÉE

(30) Priorität: 11.12.2014 DE 102014225553
(43) Veröffentlichungstag der Anmeldung: 18.10.2017
(73) Patentinhaber: Henkel AG & Co. KGaA, 40589 Düsseldorf (DE)
(72) Erfinder: MÜLLER, Burkhard, 40221 Düsseldorf (DE); KNAPPE, Thorsten, 22869 Schenefeld (DE)
(86) Internationale Anmeldenummer: PCT/EP2015/076539
(87) Internationale Veröffentlichungsnummer: WO 2016/091533

(56) Entgegenhaltungen:
- GB-A- 2 207 443
- US-A- 3 167 478
- US-A- 5 723 433

## Beschreibung

Die Anmeldung betrifft das technische Fachgebiet der Färbung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur Farbveränderung keratinhaltiger Fasern Gegenstand der vorliegenden Anmeldung.

Eine ansprechend aussehende Frisur wird heute allgemein als unverzichtbarer Teil eines gepflegten Äußeren angesehen. Um eine solche ansprechende Frisur zu realisieren werden die Haare kosmetischen Behandlungsverfahren unterworfen, die von der Reinigung mittels eines Shampoos bis zur permanenten Verformung mittels chemisch/thermischer Verfahren oder der permanenten oxidativen Farbaufhellung reichen. Zur Färbung der Haare stehen zeitlich begrenzt haltbare direktziehende Farbstoffe und Oxidationsfarbstoffe zur Verfügung. Diese Haarfarbstoffe werden in der Regel in Form flüssiger oder schaumförmiger Zubereitungen auf das Haar aufgebracht. Als Hilfsmittel bei der Applikation der Farbstoffzubereitungen werden beispielsweise Pinsel oder Schaumdispenser eingesetzt.

Zu den Schaumdispensern zählen insbesondere die Pumpsprays oder Aerosolsprays, mit deren Hilfe die kosmetischen Zubereitungen entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung oder Aufschäumung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel.

Aufgabe der vorliegenden Erfindung war es daher, spezifische haarkosmetische Zubereitungen zur Färbung keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens eine gute kosmetische Wirkung zu erzielen. Es hat sich gezeigt, dass zur Lösung dieser Aufgabe aus der Vielzahl der bekannten haarkosmetisch wirksamen Zubereitungen insbesondere lösungsmittelhaltige Zubereitungen mit spezifischen Gewichtsanteilen an Oxidationsfarbstoffvorprodukten geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt;
b) eine Vorrichtung zur Entspannungsverdampfung der kosmetischen Zubereitung a). Die erfindungsgemäße kosmetische Zubereitung enthält als ersten wesentlichen Bestandteil 25 bis 60 Gew.-% mindestens eines polaren Lösungsmittels a1). Entsprechende Mittel zeichnen sich durch eine gute kosmetische Wirkung bei gleichzeitig guter Applizierbarkeit aus.

Ein zweiter wesentlicher Bestandteil erfindungsgemäßer kosmetischer Zusammensetzungen ist das Oxidationsfarbstoffvorprodukt a2). Für die Herstellung und Lagerung wie für die kosmetischen Eigenschaften der kosmetischen Zubereitung a) hat es sich als vorteilhaft erwiesen, wenn der Gewichtsanteil des Oxidationsfarbstoffvorprodukts a2) am Gesamtgewicht der kosmetischen Zubereitung a) 0,003 bis 6,0 Gew.-% beträgt.

Unter Oxidationsfarbstoffvorprodukten sind erfindungsgemäß haarfarbverändernde Mittel zu verstehen, die durch Oxidation von Oxidationsfarbstoffvorprodukten eine dauerhafte Färbung der Fasern bewirken. Unter der Bezeichnung Oxidationsfarbstoffvorprodukt werden sogenannte Entwicklerkomponenten und Kupplerkomponenten zusammengefasst. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck p₀, zum Beispiel den umgebenden Luftdruck (p₀ = 1,000 bar), entlastet werden kann, wodurch mindestens teilweise eine Verdampfung der kosmetischen Zubereitung a) erreicht wird.

Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Zusammenfassend verfügt eine bevorzugte Vorrichtung zur Entspannungsverdampfung über
b1) einen mittels eines Ventils b2) zu verschließenden und zu öffnenden Behälter b1), welcher der geschlossenen Innenraum definiert, in dem die kosmetische Zubereitung aufgenommen werden kann,
b3) eine Heizvorrichtung b3), welche es ermöglicht eine in dem Behälter b1) befindliche kosmetische Zubereitung zu erhitzen.

Dabei ermöglicht eine zusätzliche Düse b4) eine Zerstäubung der aus dem Behälter entweichenden kosmetischen Zubereitung a). Alternativ zu einem Ventil b2) kann auch ein vergleichbar wirkendes Schließelement, welches eine zugehörige Öffnung im Behälter durch entsprechende Positionsänderung verschließen oder freigeben kann, zum Einsatz kommen.

Die kosmetische Zubereitung a) ist vorzugsweise flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverfahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol, Isopropanol und Wasser erwiesen, welche aus diesem Grund als polare Lösungsmittel a1) bevorzugt werden.

Besonders bevorzugte polare Lösungsmittel a1) oder Lösungsmittelsysteme sind dadurch gekennzeichnet, dass der Gewichtsanteil von Wasser am Gesamtgewicht des polaren Lösungsmittels a1) mehr als 80 Gew.-%, vorzugsweise mehr als 85 Gew.-% und insbesondere mehr als 90 Gew.-% beträgt.

Die kosmetischen Zubereitung a) kann als Farbstoffvorprodukte Oxidationsfarbstoffvorprodukte vom Entwickler- und/oder Kuppler-Typ, und Vorstufen naturanaloger Farbstoffe, wie Indol- und Indolin-Derivate, sowie Mischungen von Vertretern dieser Gruppen enthalten.

Als Oxidationsfarbstoffvorprodukt wird erfindungsgemäß alternativ p-Phenyldiamin oder eines seiner Derivate, Aminophenol oder eines seiner Derivate oder Resorcin oder eines seiner Derivate eingesetzt.

Im Rahmen einer ersten Ausführungsform der vorliegenden Erfindung enthalten die kosmetischen Zubereitungen a) mindestens ein Oxidationsfarbstoffvorprodukt vom Entwickler- und/oder Kupplertyp.

Als Entwicklerkomponente kann p-Phenylendiamin bzw. ein p-Phenylendiaminderivat oder eines seiner physiologisch verträglichen Salze eingesetzt werden. einzusetzen. Besonders bevorzugte p-Phenylendiamine sind ausgewählt aus p-Phenylendiamin, p-Toluylendiamin, 2-Chlorp-phenylendiamin, N,N-Bis-(2-hydroxyethyl)-p-phenylendiamin und N-Phenyl-p-phenylendiamin, 2-(2-Hydroxyethyl)-p-phenylendiamin, N-(4-Amino-3-methylphenyl)-N-[3-(1H-imidazol-1-yl)propyl]amin sowie ihren physiologisch verträglichen Salzen. Besonders bevorzugte kosmetische Produkte sind dadurch gekennzeichnet, dass die kosmetische Zubereitung a) mindestens ein Oxidationsfarbstoffvorprodukt vom Entwicklertyp, vorzugsweise mindestens eine Verbindung aus der Gruppe p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin und/oder deren physiologisch verträgliche Salze enthält.

Als Entwicklerkomponente kann ein p-Aminophenolderivat oder eines seiner physiologisch verträglichen Salze eingesetzt werden. Bevorzugte p-Aminophenole sind insbesondere p-Aminophenol, N-Methyl-p-aminophenol, und 4-Amino-3-methylphenol sowie deren physiologisch verträgliche Salze. Ferner kann die Entwicklerkomponente ausgewählt sein aus o-Aminophenol und seinen Derivaten, wie beispielsweise 2-Amino-5-methylphenol und dessen physiologisch verträglichen Salzen.

Weiterhin kann die kosmetische Zubereitung a) mindestens Resorcin oder eines seiner Derivate als Kupplerkomponente enthalten.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5- und 2,7-Dihydroxynaphthalin, 1-Acetoxy-2-methoxynaphthalin, Resorcin, 4-Chlor-resorcin und 2-Amino-3-hydroxypyridin und deren physiologisch verträgliche Salze.

Der Einsatz von Aminophenol bzw. seiner Derivate oder ihrer physiologisch verträglichen Salze als Oxidationsfarbstoffvorprodukt ist erfindungsgemäß bevorzugt.

Bevorzugte Kupplerkomponenten sind
(A) m-Aminophenol und dessen Derivate wie beispielsweise 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivate, beispielsweise 2-Amino-5-ethylphenol,
(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}amino)ethanol,
(D) o-Diaminobenzol und dessen Derivate,
(E) Di- beziehungsweise Trihydroxybenzolderivate wie beispielsweise 2-Methylresorcin und 1,2,4-Trihydroxybenzol,
(F) Pyridinderivate wie beispielsweise 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxypyridin,
(G) Naphthalinderivate wie beispielsweise 1-Naphthol und 2-Methyl-1-naphthol,
(H) Morpholinderivate wie beispielsweise 6-Hydroxybenzomorpholin,
(I) Chinoxalinderivate,
(J) Pyrazolderivate wie beispielsweise 1-Phenyl-3-methylpyrazol-5-on,
(K) Indolderivate wie beispielsweise 6-Hydroxyindol,
(L) Pyrimidinderivate oder
(M) Methylendioxybenzolderivate wie beispielsweise 1-(2'-Hydroxyethyl)-amino-3,4-methylendioxybenzol
sowie deren physiologisch verträgliche Salze.

Besonders bevorzugte alternative kosmetische Produkte sind dadurch gekennzeichnet, dass die kosmetische Zubereitung a)
- mindestens ein Oxidationsfarbstoffvorprodukt vom Kupplertyp, vorzugsweise mindestens eine Verbindung aus der Gruppe 3-Aminophenol, 5-Amino-2-methylphenol, 3-Amino-2-chlor-6-methylphenol, 2,4-Dichlor-3-aminophenol und/oder deren physiologisch verträgliche Salze enthält;
- mindestens eine Verbindung aus der Gruppe 2-(2,4-Diaminophenoxy)ethanol, 1,3-Bis(2,4-diaminophenoxy)propan, 1-Methoxy-2-amino-4-(2-hydroxyethylamino)benzol, 2,6-Bis(2'-hydroxyethylamino)-1-methylbenzol und/oder deren physiologisch verträgliche Salze enthält;
- mindestens eine Verbindung aus der Gruppe Resorcin, 2-Methylresorcin und 4-Chlorresorcin enthält;
- mindestens eine Verbindung aus der Gruppe 2-Amino-3-hydroxypyridin, 3-Amino-2-methylamino-6-methoxypyridin, 2,6-Dihydroxy-3,4-dimethylpyridin und/oder deren physiologisch verträgliche Salze enthält;
- mindestens eine Verbindung aus der Gruppe 2-Naphthol und 2,7-Dihydroxynaphthalin enthält.

Ganz besonders bevorzugte Kupplerkomponenten sind 1-Naphthol, 1,5- und 2,7-Dihydroxynaphthalin, 5-Amino-2-methylphenol, 2-Amino-3-hydroxypyridin, Resorcin, 4-Chlorresorcin, 2-Methylresorcin und 2,6-Dihydroxy-3,4-dimethylpyridin und deren physiologisch verträgliche Salze.

Der Einsatz von Resorcin bzw. seiner Derivate als Oxidationsfarbstoffvorprodukt ist erfindungsgemäß bevorzugt.

Die kosmetische Zubereitung a) enthält die Oxidationsfarbstoffvorprodukte vorzugsweise in Mengen von 0,003 bis 5,0 Gew.-%, bevorzugt 0,003 bis 3,0 Gew.-%, jeweils bezogen auf das Gesamtgewicht der kosmetischen Zubereitung a). Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Ein besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt; wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung a), umfassend
   - einen mittels eines Ventils b2 zu verschließenden und zu öffnenden Behälter b1),
   - eine Heizvorrichtung, welche es ermöglicht ein in dem Behälter b1) befindliche kosmetische Zubereitung zu erhitzen,
   - eine Düse b4), welche eine Zerstäubung der kosmetischen Zubereitung a) ermöglicht.

Ein besonders bevorzugter Gegenstand der vorliegenden Erfindung ist mit anderen Worten ein kosmetisches Produkt, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt; wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung a), wobei die Vorrichtung zur Entspannungsverdampfung einen Behälter b1) und eine Heizvorrichtung b3 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b3) unter Druckerhöhung erhitzt werden kann,
   - die erhitzte kosmetische Zubereitung a) aus dem Innenraum des Behälters b1) unter Druckminderung in die Umgebung entspannt werden kann.

Der Behälter b1), in welchem die kosmetische Zubereitung erhitzt wird, ist dabei in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung b2, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Ein weiterer besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt; wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   oder Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt; wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   oder Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt; wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   oder Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst.

Bevorzugt werden weiterhin kosmetische Produkte, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt; wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   oder Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Zusammenfassend ist ein besonders bevorzugter Gegenstand der vorliegenden Erfindung daher ein kosmetisches Produkt, umfassend
a) eine kosmetische Zubereitung, enthaltend bezogen auf ihr Gesamtgewicht
   a1) 25 bis 60 Gew.-% polares Lösungsmittel;
   a2) 0,003 bis 6,0 Gew.-% Oxidationsfarbstoffvorprodukt; wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   oder Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den beiden zuvor beschriebenen Bestandteilen a1) und a2) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Ein erster optionaler Bestandteil erfindungsgemäßer kosmetischer Zubereitungen sind die kationischen Polymere a3). Für die Herstellbarkeit und kosmetischer Wirkung der kosmetischen Zubereitung a) hat es sich als vorteilhaft erwiesen, wenn die kosmetische Zubereitung a), bezogen auf ihr Gesamtgewicht 0,2 bis 7,0 Gew.-%, vorzugsweise 0,5 bis 5,0 Gew.-% und insbesondere 1,0 bis 4,0 Gew.-% kationisches Polymer a3) enthält.

Die Gruppe der kationischen Polymere a3) umfasst insbesondere die kationische Polymere mit den INCI Bezeichnungen Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polyquaternium-68 und Polyquaternium-69. Besonders bevorzugt ist der Einsatz von kationischen Polymeren mit der INCI-Bezeichnung Polyquaternium-7, Polyquaternium-22 und Polyquaternium-39.

Eine zweite Gruppe optionaler Bestandteile der kosmetischen Zubereitungen a) bilden die zwitterionischen Tenside a4), deren Gewichtsanteil am Gesamtgewicht der kosmetischen Zubereitung vorzugsweise 0,1 bis 20 Gew.-%, vorzugsweise 1,0 bis 16 Gew.-% und insbesondere 5,0 bis 12 Gew.-% beträgt.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat. Kosmetische Produkte, dadurch gekennzeichnet, dass das zwitterionische Tensid a4) aus der Gruppe der Substanzen mit der INCI-Bezeichung Amidopropylbetain, vorzugsweise aus der Gruppe der Substanzen mit den INCI-Bezeichnungen Amidopropylbetain und Coco Betain ausgewählt ist, sind aufgrund ihrer vorteilhaften Eigenschaften besonders bevorzugt.

Eine dritte Gruppe bevorzugter optionaler Bestandteile der kosmetischen Zubereitung bilden die nichtionischen Tenside. Der Gewichtsanteil der nichtionischen Tenside am Gesamtgewicht der kosmetischen Zubereitung beträgt vorzugsweise 0,1 bis 6,0 Gew.-%, vorzugsweise 0,2 bis 5,0 Gew.-% und insbesondere 0,5 bis 4,0 Gew.-%.

Als bevorzugte nichtionische Tenside haben sich Alkylenoxid-Anlagerungsprodukte an gesättigte lineare Fettalkohole, Fettsäureester und Fettsäuren mit jeweils 2 bis 80 Mol Ethylenoxid und/oder 1 bis 5 Mol Propylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Dabei hat es sich als besonders vorteilhaft erwiesen, wenn das weitere nichtionische Tensid einen HLB-Wert oberhalb von 10, vorzugsweise oberhalb von 14 aufweist. Dazu ist notwendig, dass das nichtionische Tensid einen ausreichend hohen Ethoxylierungsgrad aufweist.

Eine weitere Ausführungsform des ersten Erfindungsgegenstands ist daher dadurch gekennzeichnet, dass das die kosmetische Zubereitung a) als nichtionisches Tensid mindestens ein ethoxyliertes Tensid mit mindestens 30 Ethylenoxideinheiten enthält.

Neben den entsprechend ethoxylierten Fettalkoholen sind erfindungsgemäß insbesondere die Anlagerungsprodukte von 30 bis 60 Mol Ethylenoxid an Rizinusöl und gehärtetes Rizinusöl besonders geeignet. Beispiele für solche geeigneten Tenside tragen die INCI-Bezeichnungen Steareth-30. Ceteareth- 30, Oleth-30, Ceteareth-50 oder PEG-40 Hydrogenated Castor Oil und PEG-60 Hydrogenated Castor Oil.

Das oder die zuvor genannten ethoxylierte(n) nichtionische(n) Tensid(e) kann (können) in einer bevorzugten Ausführungsform auch als Mischung mit Fettalkoholen und/oder ionischen Tensiden in der Oxidationsmittelzubereitung B eingesetzt werden.

Bevorzugte Beispiele für solche Mischungen sind beispielsweise Mischungen von Fettalkoholen, ethoxylierten nichtionischen Tensiden, insbesondere ethoxylierten gehärteten Rizinusölen und anionischen Sulfattensiden. Ein solches Handelsprodukt wird beispielsweise von der Firma BASF unter der Bezeichnung Emulgade F angeboten (INCI-Bezeichnung: Cetearyl Alcohol, PEG-40 Castor Oil, Sodium Cetearyl Sulfate).

Kosmetisches Produkte, dadurch gekennzeichnet, dass das nichtionische Tensid a5) aus der Gruppe der alkoxylierten Fettalkohole, vorzugsweise aus der Gruppe der ethoxylierten Fettalkohole ausgewählt ist, sind erfindungsgemäß bevorzugt.

Ein vierter bevorzugter Bestandteil der kosmetischen Zubereitungen a) sind die anionischen Tenside a6). Im Hinblick auf die Applizierbarkeit und kosmetische Wirkung ist es bevorzugt, wenn die kosmetische Zubereitung a), bezogen auf ihr Gesamtgewicht, 0,1 bis 6,0 Gew.-%, vorzugsweise 0,2 bis 4,0 Gew.-% und insbesondere 0,5 bis 3,0 Gew.-% anionisches Tensid a6) enthält.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate. Besonders bevorzugt

Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Besonders bevorzugt sind C₈-C₂₀-Alkylsulfate, insbesondere Natriumcetearylsulfat und Natriumlaurylsulfat, sowie C₈-C₂₀-Alkylethersulfate mit 2 bis 12, vorzugsweise 2 bis 4 Ethylenoxidgruppen, insbesondere Natriumlaurylethersulfat (INCI: Sodium Laureth Sulfate).

Neben den zuvor beschriebenen Inhaltsstoffen a1), a2) und a3) bis a6) können die erfindungsgemäßen kosmetischen Mittel weitere Wirk-, Hilfs- und Pflegestoffe enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| polares Lösungsmittel a1) | 40 bis 80 | 45 bis 75 | 50 bis 70 | 52 | 59 |
| Oxidationsfarbstoffvorprodukt a2) | 0,01 bis 10 | 0,02 bis 8,0 | 0,03 bis 6,0 | 5,1 | 2,4 |
| optionale Additive | add 100 | add 100 | add 100 | add 100 | add 100 |

| | Formel 6 | Formel 7 | Formel 8 | Formel 9 | Formel 10 |
|---|---|---|---|---|---|
| polares Lösungsmittel a1) | 40 bis 80 | 45 bis 75 | 50 bis 70 | 52 | 59 |
| p-Phenylendiamin oder eines seiner Derivate | 0,01 bis 10 | 0,02 bis 8,0 | 0,03 bis 6,0 | 3,2 | 1,6 |
| optionale Additive | add 100 | add 100 | add 100 | add 100 | add 100 |

| | Formel 11 | Formel 12 | Formel 13 | Formel 14 | Formel 15 |
|---|---|---|---|---|---|
| polares Lösungsmittel a1) | 40 bis 80 | 45 bis 75 | 50 bis 70 | 52 | 59 |
| Aminophenol oder eines seiner Derivate | 0,01 bis 10 | 0,02 bis 8,0 | 0,03 bis 6,0 | 0,2 | 0,1 |
| optionale Additive | add 100 | add 100 | add 100 | add 100 | add 100 |

| | Formel 16 | Formel 17 | Formel 18 | Formel 19 | Formel 20 |
|---|---|---|---|---|---|
| polares Lösungsmittel a1) | 40 bis 80 | 45 bis 75 | 50 bis 70 | 52 | 59 |
| Resorcin oder eines seiner Derivate | 0,01 bis 10 | 0,02 bis 8,0 | 0,03 bis 6,0 | 0,8 | 0,7 |
| optionale Additive | add 100 | add 100 | add 100 | add 100 | add 100 |

| | Formel 21 | Formel 22 | Formel 23 | Formel 24 | Formel 25 |
|---|---|---|---|---|---|
| polares Lösungsmittel a1) | 40 bis 80 | 45 bis 75 | 50 bis 70 | 52 | 59 |
| Oxidationsfarbstoffvorprodukt a2) | 0,01 bis 10 | 0,02 bis 8,0 | 0,03 bis 6,0 | 5,1 | 2,4 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 26 | Formel 27 | Formel 28 | Formel 29 | Formel 30 |
|---|---|---|---|---|---|
| polares Lösungsmittel a1) | 40 bis 80 | 45 bis 75 | 50 bis 70 | 52 | 59 |
| p-Phenylendiamin oder eines seiner Derivate | 0,01 bis 10 | 0,02 bis 8,0 | 0,03 bis 6,0 | 3,2 | 1,6 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 31 | Formel 32 | Formel 33 | Formel 34 | Formel 35 |
|---|---|---|---|---|---|
| polares Lösungsmittel a1) | 40 bis 80 | 45 bis 75 | 50 bis 70 | 52 | 59 |
| Aminophenol oder eines seiner Derivate | 0,01 bis 10 | 0,02 bis 8,0 | 0,03 bis 6,0 | 0,2 | 0,1 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 36 | Formel 37 | Formel 38 | Formel 39 | Formel 40 |
|---|---|---|---|---|---|
| polares Lösungsmittel a1) | 40 bis 80 | 45 bis 75 | 50 bis 70 | 52 | 59 |
| Resorcin oder eines seiner Derivate | 0,01 bis 10 | 0,02 bis 8,0 | 0,03 bis 6,0 | 0,8 | 0,7 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 41 | Formel 42 | Formel 43 | Formel 44 | Formel 45 |
|---|---|---|---|---|---|
| polares Lösungsmittel a1) | 40 bis 80 | 45 bis 75 | 50 bis 70 | 52 | 59 |
| Oxidationsfarbstoffvorprodukt a2) | 0,01 bis 10 | 0,02 bis 8,0 | 0,03 bis 6,0 | 5,1 | 2,4 |
| zwitterionisches Tensid a4) | 0,1 bis 20 | 1,0 bis 16 | 5,0 bis 12 | 1,8 | 10 |
| optionale Additive | add 100 | add 100 | add 100 | add 100 | add 100 |

| | Formel 46 | Formel 47 | Formel 48 | Formel 49 | Formel 50 |
|---|---|---|---|---|---|
| polares Lösungsmittel a1) | 40 bis 80 | 45 bis 75 | 50 bis 70 | 52 | 59 |
| p-Phenylendiamin oder eines seiner Derivate | 0,01 bis 10 | 0,02 bis 8,0 | 0,03 bis 6,0 | 3,2 | 1,6 |
| zwitterionisches Tensid a4) | 0,1 bis 20 | 1,0 bis 16 | 5,0 bis 12 | 1,8 | 10 |
| optionale Additive | add 100 | add 100 | add 100 | add 100 | add 100 |

| | Formel 51 | Formel 52 | Formel 53 | Formel 54 | Formel 55 |
|---|---|---|---|---|---|
| polares Lösungsmittel a1) | 40 bis 80 | 45 bis 75 | 50 bis 70 | 52 | 59 |
| Aminophenol oder eines seiner Derivate | 0,01 bis 10 | 0,02 bis 8,0 | 0,03 bis 6,0 | 0,2 | 0,1 |
| zwitterionisches Tensid a4) | 0,1 bis 20 | 1,0 bis 16 | 5,0 bis 12 | 1,8 | 10 |
| optionale Additive | add 100 | add 100 | add 100 | add 100 | add 100 |

| | Formel 56 | Formel 57 | Formel 58 | Formel 59 | Formel 60 |
|---|---|---|---|---|---|
| polares Lösungsmittel a1) | 40 bis 80 | 45 bis 75 | 50 bis 70 | 52 | 59 |
| Resorcin oder eines seiner Derivate | 0,01 bis 10 | 0,02 bis 8,0 | 0,03 bis 6,0 | 0,8 | 0,7 |
| zwitterionisches Tensid a4) | 0,1 bis 20 | 1,0 bis 16 | 5,0 bis 12 | 1,8 | 10 |
| optionale Additive | add 100 | add 100 | add 100 | add 100 | add 100 |

| | Formel 61 | Formel 62 | Formel 63 | Formel 64 | Formel 65 |
|---|---|---|---|---|---|
| polares Lösungsmittel a1) | 40 bis 80 | 45 bis 75 | 50 bis 70 | 52 | 59 |
| Oxidationsfarbstoffvorprodukt a2) | 0,01 bis 10 | 0,02 bis 8,0 | 0,03 bis 6,0 | 5,1 | 2,4 |
| nichtionisches Tensid a5) | 0,1 bis 6,0 | 0,2 bis 5,0 | 0,5 bis 4,0 | 1,2 | 3,0 |
| optionale Additive | add 100 | add 100 | add 100 | add 100 | add 100 |

| | Formel 66 | Formel 67 | Formel 68 | Formel 69 | Formel 70 |
|---|---|---|---|---|---|
| polares Lösungsmittel a1) | 40 bis 80 | 45 bis 75 | 50 bis 70 | 52 | 59 |
| p-Phenylendiamin oder eines seiner Derivate | 0,01 bis 10 | 0,02 bis 8,0 | 0,03 bis 6,0 | 3,2 | 1,6 |
| nichtionisches Tensid a5) | 0,1 bis 6,0 | 0,2 bis 5,0 | 0,5 bis 4,0 | 1,2 | 3,0 |
| optionale Additive | add 100 | add 100 | add 100 | add 100 | add 100 |

| | Formel 71 | Formel 72 | Formel 73 | Formel 74 | Formel 75 |
|---|---|---|---|---|---|
| polares Lösungsmittel a1) | 40 bis 80 | 45 bis 75 | 50 bis 70 | 52 | 59 |
| Aminophenol oder eines seiner Derivate | 0,01 bis 10 | 0,02 bis 8,0 | 0,03 bis 6,0 | 0,2 | 0,1 |
| nichtionisches Tensid a5) | 0,1 bis 6,0 | 0,2 bis 5,0 | 0,5 bis 4,0 | 1,2 | 3,0 |
| optionale Additive | add 100 | add 100 | add 100 | add 100 | add 100 |

| | Formel 76 | Formel 77 | Formel 78 | Formel 79 | Formel 80 |
|---|---|---|---|---|---|
| polares Lösungsmittel a1) | 40 bis 80 | 45 bis 75 | 50 bis 70 | 52 | 59 |
| Resorcin oder eines seiner Derivate | 0,01 bis 10 | 0,02 bis 8,0 | 0,03 bis 6,0 | 0,8 | 0,7 |
| nichtionisches Tensid a5) | 0,1 bis 6,0 | 0,2 bis 5,0 | 0,5 bis 4,0 | 1,2 | 3,0 |
| optionale Additive | add 100 | add 100 | add 100 | add 100 | add 100 |

| | Formel 81 | Formel 82 | Formel 83 | Formel 84 | Formel 85 |
|---|---|---|---|---|---|
| polares Lösungsmittel a1) | 40 bis 80 | 45 bis 75 | 50 bis 70 | 52 | 59 |
| Oxidationsfarbstoffvorprodukt a2) | 0,01 bis 10 | 0,02 bis 8,0 | 0,03 bis 6,0 | 5,1 | 2,4 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 86 | Formel 87 | Formel 88 | Formel 89 | Formel 90 |
|---|---|---|---|---|---|
| polares Lösungsmittel a1) | 40 bis 80 | 45 bis 75 | 50 bis 70 | 52 | 59 |
| p-Phenylendiamin oder eines seiner Derivate | 0,01 bis 10 | 0,02 bis 8,0 | 0,03 bis 6,0 | 3,2 | 1,6 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 91 | Formel 92 | Formel 93 | Formel 94 | Formel 95 |
|---|---|---|---|---|---|
| polares Lösungsmittel a1) | 40 bis 80 | 45 bis 75 | 50 bis 70 | 52 | 59 |
| Aminophenol oder eines seiner Derivate | 0,01 bis 10 | 0,02 bis 8,0 | 0,03 bis 6,0 | 0,2 | 0,1 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 96 | Formel 97 | Formel 98 | Formel 99 | Formel 100 |
|---|---|---|---|---|---|
| polares Lösungsmittel a1) | 40 bis 80 | 45 bis 75 | 50 bis 70 | 52 | 59 |
| Resorcin oder eines seiner Derivate | 0,01 bis 10 | 0,02 bis 8,0 | 0,03 bis 6,0 | 0,8 | 0,7 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 101 | Formel 102 | Formel 103 | Formel 104 | Formel 105 |
|---|---|---|---|---|---|
| polares Lösungsmittel a1) | 40 bis 80 | 45 bis 75 | 50 bis 70 | 52 | 59 |
| Oxidationsfarbstoffvorprodukt a2) | 0,01 bis 10 | 0,02 bis 8,0 | 0,03 bis 6,0 | 5,1 | 2,4 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 106 | Formel 107 | Formel 108 | Formel 109 | Formel 110 |
|---|---|---|---|---|---|
| polares Lösungsmittel a1) | 40 bis 80 | 45 bis 75 | 50 bis 70 | 52 | 59 |
| p-Phenylendiamin oder eines seiner Derivate | 0,01 bis 10 | 0,02 bis 8,0 | 0,03 bis 6,0 | 3,2 | 1,6 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 111 | Formel 112 | Formel 113 | Formel 114 | Formel 115 |
|---|---|---|---|---|---|
| polares Lösungsmittel a1) | 40 bis 80 | 45 bis 75 | 50 bis 70 | 52 | 59 |
| Aminophenol oder eines seiner Derivate | 0,01 bis 10 | 0,02 bis 8,0 | 0,03 bis 6,0 | 0,2 | 0,1 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 116 | Formel 117 | Formel 118 | Formel 119 | Formel 120 |
|---|---|---|---|---|---|
| polares Lösungsmittel a1) | 40 bis 80 | 45 bis 75 | 50 bis 70 | 52 | 59 |
| Resorcin oder eines seiner Derivate | 0,01 bis 10 | 0,02 bis 8,0 | 0,03 bis 6,0 | 0,8 | 0,7 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 121 | Formel 122 | Formel 123 | Formel 124 | Formel 125 |
|---|---|---|---|---|---|
| Wasser | 40 bis 80 | 45 bis 75 | 50 bis 70 | 52 | 59 |
| Oxidationsfarbstoffvorprodukt a2) | 0,01 bis 10 | 0,02 bis 8,0 | 0,03 bis 6,0 | 5,1 | 2,4 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 126 | Formel 127 | Formel 128 | Formel 129 | Formel 130 |
|---|---|---|---|---|---|
| Wasser | 40 bis 80 | 45 bis 75 | 50 bis 70 | 52 | 59 |
| p-Phenylendiamin oder eines seiner Derivate | 0,01 bis 10 | 0,02 bis 8,0 | 0,03 bis 6,0 | 3,2 | 1,6 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 131 | Formel 132 | Formel 133 | Formel 134 | Formel 135 |
|---|---|---|---|---|---|
| Wasser | 40 bis 80 | 45 bis 75 | 50 bis 70 | 52 | 59 |
| Aminophenol oder eines seiner Derivate | 0,01 bis 10 | 0,02 bis 8,0 | 0,03 bis 6,0 | 0,2 | 0,1 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 136 | Formel 137 | Formel 138 | Formel 139 | Formel 140 |
|---|---|---|---|---|---|
| Wasser | 40 bis 80 | 45 bis 75 | 50 bis 70 | 52 | 59 |
| Resorcin oder eines seiner Derivate | 0,01 bis 10 | 0,02 bis 8,0 | 0,03 bis 6,0 | 0,8 | 0,7 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 20 | 1,0 bis 16 | 5,0 bis 12 | 1,8 | 10 |
| nichtionisches Tensid a5) | 0,1 bis 6,0 | 0,2 bis 5,0 | 0,5 bis 4,0 | 1,2 | 3,0 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

Ganz besonders bevorzugte kosmetische Zubereitungen enthalten neben den zuvor beschriebenen Bestandteilen a1) bis a6) nur geringe Mengen weiterer Wirk- und Hilfsstoffe. Kosmetische Zubereitungen, dadurch gekennzeichnet, dass der Gewichtsanteil der Bestandteile a1), a2) sowie, falls vorhanden, der optionalen Bestandteilen a3) bis a6) am Gesamtgewicht der kosmetischen Zubereitung mindestens 86 Gew.-%, vorzugsweise mindestens 90 Gew.-% und insbesondere mindestens 94 Gew.-% beträgt, sind aufgrund ihrer einfachen Herstellbarkeit und guten kosmetischen Wirkung besonders bevorzugt.

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 25 bis 60 Gew.-% polares Lösungsmittel;
a2) 0,003 bis 6,0 Gew.-% Oxidationsfarbstoffvorprodukt;
als Prozessgut in einer anspruchsgemäßen

Vorrichtung zur Entspannungsverdampfung. Gegenstand der vorliegenden Anmeldung ist zudem die Verwendung eines erfindungsgemäßen Produktes zur Beaufschlagung keratinhaltiger Fasern, insbesondere menschlicher Haare, mit einer kosmetischen Zubereitung a) bzw. zur Farbveränderung keratinhaltiger Fasern, insbesondere menschlicher Haare.

Ein Verfahren zur Farbveränderung keratinhaltiger Fasern, insbesondere menschlicher Haare, bei welchem die keratinhaltigen Fasern mittels einer anspruchsgemäßen Vorrichtung zur Entspannungsverdampfung mit
einer kosmetischen Zubereitung a) enthaltend bezogen auf ihr Gesamtgewicht
a1) 25 bis 60 Gew.-% polares Lösungsmittel;
a2) 0,003 bis 6,0 Gew.-% Oxidationsfarbstoffvorprodukt;
   beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Ein bevorzugter Anmeldungsgegenstand ist ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 25 bis 60 Gew.-% polares Lösungsmittel;
a2) 0,003 bis 6,0 Gew.-% Oxidationsfarbstoffvorprodukt;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

Bezüglich weiterer bevorzugter Ausführungsformen der erfindunsgemäßen Verwendungen und des erfindungsgemäßen Verfahrens gilt mutatis mutandis das zu den erfindungsgemäßen kosmetischen Zubereitungen a) und zu der Vorrichtung zur Entspannungsverdampfung b) Gesagte.

## Patentansprüche

1. Kosmetisches Produkt, umfassend
a) eine kosmetische Zubereitung (a), enthaltend bezogen auf ihr Gesamtgewicht
a1) 25 bis 60 Gew.-% polares Lösungsmittel;
a2) 0,003 bis 6,0 Gew.-% Oxidationsfarbstoffvorprodukt, wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
b) eine Vorrichtung zur Entspannungsverdampfung der kosmetischen Zubereitung (a) mit
b1) einem Behälter (b1), welcher einen geschlossenen Innenraum definiert, in dem die kosmetische Zubereitung (a) aufgenommen werden kann,
b2) einem Ventil oder einem vergleichbar wirkenden Schließelement, um den wenigstens anteilsweise mit der kosmetischen Zubereitung (a) befüllten Innenraum des Behälters (b1) zu verschließen und zu öffnen,
b3) einer Heizvorrichtung, um die in dem geschlossenen Innenraum des Behälters (b1) befindliche kosmetische Zubereitung (a) unter Druckerhöhung zu erhitzen, sowie die erhitzte kosmetische Zubereitung (a) aus dem Innenraum des Behälters (b1) unter Druckminderung in die Umgebung zu entspannen,
b4) einer Düse, welche eine Zerstäubung der aus dem Behälter (b1) entweichenden kosmetischen Zubereitung (a) ermöglicht.

2. Kosmetisches Produkt nach einem der vorherigen Ansprüche, **dadurch gekennzeichnet, dass** das polare Lösungsmittel (a1) ausgewählt ist aus der Gruppe Wasser und Ethanol, vorzugsweise Wasser.

3. Kosmetisches Produkt nach einem der vorherigen Ansprüche, **dadurch gekennzeichnet, dass** die kosmetische Zubereitung (a) bezogen auf ihr Gesamtgewicht 0,2 bis 7,0 Gew.%, vorzugsweise 0,5 bis 5,0 Gew.-% und insbesondere 1,0 bis 4,0 Gew.-% kationisches Polymer a3) enthält.

4. Kosmetisches Produkt nach einem der vorherigen Ansprüche, **dadurch gekennzeichnet, dass** die kosmetische Zubereitung (a) bezogen auf ihr Gesamtgewicht 0,1 bis 20 Gew.-%, vorzugsweise 1,0 bis 16 Gew.-% und insbesondere 5,0 bis 12 Gew.-% zwitterionisches Tensid enthält.

5. Verwendung einer kosmetischen Zubereitung (a) enthaltend, bezogen auf ihr Gesamtgewicht,
a) eine kosmetische Zubereitung (a), enthaltend bezogen auf ihr Gesamtgewicht
a1) 25 bis 60 Gew.-% polares Lösungsmittel;
a2) 0,003 bis 6,0 Gew.-% Oxidationsfarbstoffvorprodukt, wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
als Prozessgut in einer Vorrichtung zur Entspannungsverdampfung der kosmetischen Zubereitung (a) mit
b1) einem Behälter (b1), welcher einen geschlossenen Innenraum definiert, in dem die kosmetische Zubereitung (a) aufgenommen werden kann,
b2) einem Ventil oder einem vergleichbar wirkenden Schließelement, um den wenigstens anteilsweise mit der kosmetischen Zubereitung (a) befüllten Innenraum des Behälters (b1) zu verschließen und zu öffnen,
b3) einer Heizvorrichtung, um die in dem geschlossenen Innenraum des Behälters (b1) befindliche kosmetische Zubereitung (a) unter Druckerhöhung zu erhitzen, sowie die erhitzte kosmetische Zubereitung (a) aus dem Innenraum des Behälters (b1) unter Druckminderung in die Umgebung zu entspannen,
b4) einer Düse, welche eine Zerstäubung der aus dem Behälter (b1) entweichenden kosmetischen Zubereitung (a) ermöglicht.

6. Verwendung eines Produkts nach einem der Ansprüche 1 bis 4 zur Beaufschlagung keratinhaltiger Fasern, insbesondere menschlicher Haare, mit einer kosmetischen Zubereitung (a).

7. Verwendung eines Produkts nach einem der Ansprüche 1 bis 4 zur Farbveränderung keratinhaltiger Fasern, insbesondere menschlicher Haare.

8. Verfahren zur Farbveränderung keratinhaltiger Fasern, insbesondere menschlicher Haare, bei welchem die keratinhaltigen Fasern mittels einer Vorrichtung zur Entspannungsverdampfung einer kosmetischen Zubereitung (a) mit
b1) einem Behälter (b1), welcher einen geschlossenen Innenraum definiert, in dem die kosmetische Zubereitung (a) aufgenommen werden kann,
b2) einem Ventil oder einem vergleichbar wirkenden Schließelement, um den wenigstens anteilsweise mit der kosmetischen Zubereitung (a) befüllten Innenraum des Behälters (b1) zu verschließen und zu öffnen,
b3) einer Heizvorrichtung, um die in dem geschlossenen Innenraum des Behälters (b1) befindliche kosmetische Zubereitung (a) unter Druckerhöhung zu erhitzen, sowie die erhitzte kosmetische Zubereitung (a) aus dem Innenraum des Behälters (b1) unter Druckminderung in die Umgebung zu entspannen,
b4) einer Düse, welche eine Zerstäubung der aus dem Behälter (b1) entweichenden kosmetischen Zubereitung (a) ermöglicht,
mit einer kosmetischen Zubereitung (a) enthaltend bezogen auf ihr Gesamtgewicht
a1) 25 bis 60 Gew.-% polares Lösungsmittel;
a2) 0,003 bis 6,0 Gew.-% Oxidationsfarbstoffvorprodukt, wobei als Oxidationsfarbstoffvorprodukt alternativ p-Phenyldiamin oder eines seiner Derivate, Aminophenol oder eines seiner Derivate oder Resorcin oder eines seiner Derivate eingesetzt wird;
beaufschlagt werden.

9. Verfahren nach Anspruch 8, **dadurch gekennzeichnet, dass**
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung (a) in einen Behälter (b1) überführt wird;
- nachfolgend der Zugang zwischen Vorratsbehälter und Behälter (b1) durch ein Bauteil zur Durchflussregelung, mittels dessen der Durchfluss der kosmetischen Zubereitung (a) aus dem Vorratsbehälter in den Behälter (b1) unterbrochen werden kann, unterbrochen wird;
- nachfolgend die in dem gegen die Umgebung abgeschlossenen Behälter (b1) befindliche kosmetische Zubereitung (a) mittels einer Heizvorrichtung erhitzt wird, so dass der Druck im Inneren des Behälters (b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter (b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter (b1) befindlichen kosmetischen Zubereitung (a) aus dem Behälter in die Umgebung unter Minderung des zum Zeitpunkt der Behälteröffnung in dem Behälter (b1) herrschenden Drucks, entspannt wird.

## Claims

1. A cosmetic product, comprising
a) a cosmetic preparation (a) containing, based on the total weight thereof,
a1) 25 to 60 wt.% polar solvent;
a2) 0.003 to 6.0 wt.% oxidation dye precursor, wherein p-phenyldiamine or one of its derivatives, aminophenol or one of its derivatives, or resorcinol or one of its derivatives is alternatively used as the oxidation dye precursor;
b) a device for flash evaporation of the cosmetic preparation (a), comprising
b1) a container (b1) which defines a closed interior in which the cosmetic preparation (a) can be received,
b2) a valve or a similarly functioning closure element for closing and opening the interior of the container (b1), which is at least partly filled with the cosmetic preparation (a),
b3) a heating device for heating, under increased pressure, the cosmetic preparation (a) that is located in the closed interior of the container (b1), and releasing, under reduced pressure, the heated cosmetic preparation (a) from the interior of the container (b1) into the surroundings,
b4) a nozzle which allows atomization of the cosmetic preparation (a) escaping from the container (b1).

2. The cosmetic product according to one of the preceding claims,
**characterized in that** the polar solvent (a1) is selected from the group consisting of water and ethanol, preferably water.

3. The cosmetic product according to one of the preceding claims,
**characterized in that** the cosmetic preparation (a) contains, based on the total weight thereof, 0.2 to 7.0 wt.%, preferably 0.5 to 5.0 wt.%, and in particular 1.0 to 4.0 wt.% cationic polymer a3).

4. The cosmetic product according to one of the preceding claims,
**characterized in that** the cosmetic preparation (a) contains, based on the total weight thereof, 0.1 to 20 wt.%, preferably 1.0 to 16 wt.%, and in particular 5.0 to 12 wt.%, zwitterionic surfactant.

5. The use of a cosmetic preparation (a) containing, based on the total weight thereof,
a) a cosmetic preparation (a) containing, based on the total weight thereof,
a1) 25 to 60 wt.% polar solvent;
a2) 0.003 to 6.0 wt.% oxidation dye precursor, wherein p-phenyldiamine or one of its derivatives, aminophenol or one of its derivatives, or resorcinol or one of its derivatives is alternatively used as the oxidation dye precursor;
as a process material in a device for flash evaporation of the cosmetic preparation (a), said device comprising
b1) a container (b1) which defines a closed interior in which the cosmetic preparation (a) can be received,
b2) a valve or a similarly functioning closure element for closing and opening the interior of the container (b1), which is at least partly filled with the cosmetic preparation (a),
b3) a heating device for heating, under increased pressure, the cosmetic preparation (a) that is located in the closed interior of the container (b1), and releasing, under reduced pressure, the heated cosmetic preparation (a) from the interior of the container (b1) into the surroundings,
b4) a nozzle which allows atomization of the cosmetic preparation (a) escaping from the container (b1).

6. The use of a product according to one of claims 1 to 4 for applying a cosmetic preparation (a) to keratin-containing fibers, in particular human hair.

7. The use of a product according to one of claims 1 to 4 for changing the color of keratin-containing fibers, in particular human hair.

8. A method for treating keratin-containing fibers, in particular human hair, in which method, by means of a device for flash evaporation of a cosmetic preparation (a), said device comprising
b1) a container (b1) which defines a closed interior in which the cosmetic preparation (a) can be received,
b2) a valve or a similarly functioning closure element for closing and opening the interior of the container (b1), which is at least partly filled with the cosmetic preparation (a),
b3) a heating device for heating, under increased pressure, the cosmetic preparation (a) that is located in the closed interior of the container (b1), and releasing, under reduced pressure, the heated cosmetic preparation (a) from the interior of the container (b1) into the surroundings,
b4) a nozzle which allows atomization of the cosmetic preparation (a) escaping from the container (b1),
a cosmetic preparation (a) containing, based on the total weight thereof,
a1) 25 to 60 wt.% polar solvent;
a2) 0.003 to 6.0 wt.% oxidation dye precursor, wherein p-phenyldiamine or one of its derivatives, aminophenol or one of its derivatives, or resorcinol or one of its derivatives is alternatively used as the oxidation dye precursor;
is applied to the keratin-containing fibers.

9. The method according to claim 8, **characterized in that**
- some of the cosmetic preparation (a) located in a reservoir, in the interior of which a pressure prevails that corresponds to the ambient pressure, is transferred from said reservoir into a container (b1);
- access between the reservoir and the container (b1) is subsequently interrupted using a component for flow control, by means of which component the flow of the cosmetic preparation (a) from the reservoir into the container (b1) can be interrupted;
- the cosmetic preparation (a) located in the container (b1), which is closed off from the surroundings, is subsequently heated by means of a heating device, such that the pressure inside the container (b1) increases to values above the ambient pressure, preferably to values between 1.1 and 8 bar, in particular to values between 1.2 and 4 bar;
- the container (b1) which is pressurized above the ambient pressure is subsequently opened in such a manner that the discharge of at least some, preferably at least 50 wt.%, more preferably at least 80 wt.% and in particular at least 90 wt.%, of the cosmetic preparation (a) located in the container (b1) is released from the container into the surroundings by the pressure prevailing in the container (b1) at the time at which the container is opened being reduced.

## Revendications

1. Produit cosmétique, comprenant
a) une préparation cosmétique (a) contenant, par rapport à son poids total,
a1) 65 à 75 % en poids de solvant polaire ;
a2) 0,003 à 3,0 % en poids de colorant à action directe, dans lequel, en guise de colorant à action directe, en variante, un colorant cationique, un colorant anionique ou un colorant non ionique est utilisé ;
b) un dispositif permettant la vaporisation par détente de la préparation cosmétique (a) comportant b1) un récipient (b1) qui définit un espace intérieur fermé dans lequel la préparation cosmétique (a) peut être reçue,
b2) une soupape ou un élément de fermeture à action comparable afin de fermer et d'ouvrir l'espace intérieur du récipient (b1) rempli au moins partiellement de la préparation cosmétique (a),
b3) un dispositif de chauffage afin de chauffer la préparation cosmétique (a) se trouvant dans l'espace intérieur fermé du récipient (b1) par augmentation de la pression, ainsi que de vaporiser par détente la préparation cosmétique (a) chauffée hors de l'espace intérieur du récipient (b1) et dans l'environnement par diminution de la pression,
b4) une buse permettant une atomisation de la préparation cosmétique (a) s'échappant du récipient (b1).

2. Produit cosmétique selon l'une des revendications précédentes,
**caractérisé en ce que** le solvant polaire a1) est choisi dans le groupe constitué d'eau et d'éthanol.

3. Produit cosmétique selon l'une des revendications précédentes,
**caractérisé en ce que** la préparation cosmétique (a) contient, par rapport à son poids total, 0,1 à 5,0 % en poids, de préférence 0,2 à 3,0 % en poids et en particulier 0,5 à 2,0 % en poids d'un polymère cationique a3).

4. Produit cosmétique selon l'une des revendications précédentes, **caractérisé en ce que** la préparation cosmétique (a) contient, par rapport à son poids total, 0,1 à 7,0 % en poids, de préférence 0,2 à 5,0 % en poids et en particulier 0,5 à 3,0 % en poids d'un tensioactif zwitterionique a4).

5. Utilisation d'une préparation cosmétique (a) contenant, par rapport à son poids total,
a) une préparation cosmétique (a) contenant, par rapport à son poids total,
a1) 65 à 75 % en poids de solvant polaire ;
a2) 0,003 à 3,0 % en poids de colorant à action directe, dans lequel, en guise de colorant à action directe, en variante, un colorant cationique, un colorant anionique ou un colorant non ionique est utilisé ;
en tant que matériau de processus dans un dispositif permettant la vaporisation par détente de la préparation cosmétique (a) comportant
b1) un récipient (b1) qui définit un espace intérieur fermé dans lequel la préparation cosmétique (a) peut être reçue,
b2) une soupape ou un élément de fermeture à action comparable afin de fermer et d'ouvrir l'espace intérieur du récipient (b1) rempli au moins partiellement de la préparation cosmétique (a),
b3) un dispositif de chauffage afin de chauffer la préparation cosmétique (a) se trouvant dans l'espace intérieur fermé du récipient (b1) par augmentation de la pression, ainsi que de vaporiser par détente la préparation cosmétique (a) chauffée hors de l'espace intérieur du récipient (b1) et dans l'environnement par diminution de la pression,
b4) une buse permettant une atomisation de la préparation cosmétique (a) s'échappant du récipient (b1).

6. Utilisation d'un produit selon l'une des revendications 1 à 4 permettant l'application d'une préparation cosmétique (a) sur des fibres kératiniques, en particulier sur des cheveux humains.

7. Utilisation d'un produit selon l'une des revendications 1 à 4 pour le changement de couleur de fibres kératiniques, en particulier de cheveux humains.

8. Procédé permettant le changement de couleur de fibres kératiniques, en particulier de cheveux humains, dans lequel est appliquée sur les fibres kératiniques, au moyen d'un dispositif permettant la vaporisation par détente d'une préparation cosmétique (a) comportant
b1) un récipient (b1) qui définit un espace intérieur fermé dans lequel la préparation cosmétique (a) peut être reçue,
b2) une soupape ou un élément de fermeture à action comparable afin de fermer et d'ouvrir l'espace intérieur du récipient (b1) rempli au moins partiellement de la préparation cosmétique (a),
b3) un dispositif de chauffage afin de chauffer la préparation cosmétique (a) se trouvant dans l'espace intérieur fermé du récipient (b1) par augmentation de la pression, ainsi que de vaporiser par détente la préparation cosmétique (a) chauffée hors de l'espace intérieur du récipient (b1) et dans l'environnement par diminution de la pression,
b4) une buse permettant une atomisation de la préparation cosmétique (a) s'échappant du récipient (b1),
une préparation cosmétique (a) contenant, par rapport à son poids total,
a1) 65 à 75 % en poids de solvant polaire ;
a2) 0,003 à 3,0 % en poids de colorant à action directe, dans lequel, en guise de colorant à action directe, en variante, un colorant cationique, un colorant anionique ou un colorant non ionique est utilisé.

9. Procédé selon la revendication 8, **caractérisé en ce que**
- à partir d'un réservoir de stockage à l'intérieur duquel règne une pression correspondant à la pression environnante, une fraction de la préparation cosmétique (a) se trouvant dans ledit réservoir de stockage est transférée dans un récipient (b1) ;
- l'accès entre le réservoir de stockage et le récipient (b1) est ensuite interrompu par un composant permettant la régulation du débit, au moyen duquel le débit de la préparation cosmétique a) hors du réservoir de stockage et dans le récipient (b1) peut être interrompu ;
- ensuite, la préparation cosmétique (a) se trouvant dans le récipient (b1) fermé par rapport à l'environnement est chauffée au moyen d'un dispositif de chauffage, de sorte que la pression à l'intérieur du récipient (b1) augmente jusqu'à des valeurs supérieures à la pression environnante, de préférence jusqu'à des valeurs comprises entre 1,1 et 8 bar, en particulier jusqu'à des valeurs comprises entre 1,2 et 4 bar ;
- le récipient (b1) se trouvant à une pression supérieure à la pression environnante est ensuite ouvert de manière à vaporiser par détente la sortie d'au moins une fraction, de préférence d'au moins 50 % en poids, de manière particulièrement préférée d'au moins 80 % en poids et en particulier d'au moins 90 % en poids de la préparation cosmétique (a) se trouvant dans le récipient (b1) hors du récipient (b1) et dans l'environnement, par diminution de la pression régnant dans le récipient (b1) au moment de l'ouverture du récipient.
